# EUROPEAN PATENT APPLICATION

(11) **EP 3 000 488 A1**
(43) Date of publication of application: **30.03.2016**
(21) Application number: 14306484.8
(22) Date of filing: 26.09.2014
(51) Int. Cl.: A61L 27/20, A61L 27/56, A61L 27/58

(54) **Method for preparing chitosan matrices having improved mechanical properties**

(71) Applicant: Sofradim Production, 01600 Trevoux (FR)
(72) Inventor: Ladet, Sébastien, 69300 Caluire et Cuire (FR); Vestberg, Robert, 69002 Lyon (FR); Desorme, Mylène, 69730 Genay (FR); Noorman, Camille, 69580 Sathonay Camp (FR)
(74) Representative: Delorme, Nicolas

(57) **Abstract**

The present invention relates to a method for preparing a chitosan matrix having good mechanical properties comprising the steps of
- a) preparing a solution of chitosan,
- b) pouring the solution of a) into a mould in order to form a layer,
- c) lyophilizing the layer of b) in order to obtain a matrix of chitosan,
- d) sterilizing the matrix obtained in c) by water vapor treatment.

The invention also relates to the matrices obtained and to surgical implants comprising such matrices.

The invention further relates to a method for increasing the suture retention strength of a chitosan matrix obtained by lyophilization of a chitosan solution, and to a method for for decreasing the solubilization rate in hydrochloric acid of a chitosan matrix obtained by lyophilization of a chitosan solution, comprising the step of sterilizing said matrix by a water vapor treatment.

## Description

The present invention relates to a method for preparing chitosan matrices showing good mechanical strength, lower permeability and slow solubilization rates. The invention further relates to matrices obtainable by this method and to surgical implants, for example hernia repair implants or haemostats, comprising such matrices. The invention further relates to a method for increasing the suture retention strength of chitosan matrices and to a method for decreasing the solubilisation rate of chitosan matrices.

Chitosan is a polysaccharide which results from the deacetylation of chitin. Chitin is one of the most widespread natural polysaccharides on earth and is extracted from the exoskeletons of arthropods, from the endoskeletons of cephalopods and also from fungi.

Chitosan has properties, such as biodegradability, bioresorbability, biocompatibility, non-toxicity and mechanical properties, which make it particularly advantageous for medical applications. Thus, chitosan can be incorporated into medical devices as a constituent of implants, for example in the form of matrices. Chitosan in fact offers a good compromise, given the desired properties, for matrices based implants, namely good biological compatibility and good mechanical properties.

Chitosan matrices are usually obtained by lyophilization of a chitosan solution. Lyophilization involves a first step during which a solution is frozen in a particular structure, and then a second step, during which a controlled pressure is applied in order to cause sublimation of the water present in the frozen structure. At the end of the sublimation step, only the constituents present in the initial solution remain, thus producing a porous structure.

Indeed, the porous structure of such chitosan matrices is of particular interest in the manufacture of surgical implants for many reasons. When the implant is intended to repair a tissue defect, for example in the case of a hernia in the abdominal wall, a porous structure favors cell colonization and therefore reconstruction of the biological tissue. Alternatively, when the implant is intended to be used as a haemostatic patch, in order to stop bleeding, the porous structure allows efficiently absorbing biological fluids, like blood.

Nevertheless, reinforcing implants and haemostatic patches must also show very good mechanical properties, such as a high suture retention strength. They also preferably should show slow solubilisation rates in order to avoid that the implant degrades too rapidly once in contact with biological fluids. Moreover, chitosan matrices should also have good handling characteristics.

Chitosan matrices to be used in the manufacture of surgical implants exist. Anyway, existing chitosan based matrices may show poor mechanical strength or may solubilize too rapidly when put in contact with biological fluids.

It would be interesting to provide a method for preparing chitosan matrices having improved mechanical strength, low permeability and slow solubilization rates in order to use them in the manufacture of surgical implants, such as for example hernia repair implants or haemostatic patches.

The Applicant has found that by performing a specific sterilization step on matrices obtained by lyophilization of a solution of chitosan, it was possible to obtain chitosan matrices having enhanced mechanical properties, in particular enhanced suture retention strength, while obtaining sterilized matrices at the same time.

A first aspect of the present invention is a method for preparing a chitosan matrix having good mechanical properties, in particular good suture retention strength, comprising the steps of :
- a) preparing a solution of chitosan,
- b) pouring the solution of a) into a mould in order to form a layer,
- c) lyophilizing the layer of b) in order to obtain a matrix of chitosan,
d) sterilizing the matrix obtained in c) by water vapor treatment.

The method according to the invention makes it possible to obtain sterilized chitosan matrices showing excellent mechanical properties such as suture retention strength. In particular, the method of the invention allows increasing the mechanical properties of chitosan matrices and sterilizing said matrices in a single step. The manufacturing process of chitosan matrices may therefore be rendered particularly simple. The chitosan matrices obtained by means of the method according to the invention have improved suture retention strength compared to matrices of the same chitosan composition which are not sterilized or which have been sterilized according to another sterilization method. The chitosan matrices obtained by means of the method according to the invention also show a lower permeability and a decrease in solubilisation rates compared to matrices of the same chitosan composition which are not sterilized. The chitosan matrices obtained by means of the method according to the invention therefore make it possible to obtain particularly effective surgical implants, for example for reinforcing the abdominal wall. The chitosan matrices obtained by the method of the invention are also particularly useful in the manufacture of haemostats such as haemostatic patches. The chitosan matrices obtained by the method of the invention may be used on their own or in combination with a textile or with any other element, such as for example a film, a matrix of same or different composition.

Another aspect of the invention is therefore a sterilized chitosan matrix obtainable by the method of the invention. For example, the sterilized chitosan matrix obtainable by the method of the invention has a suture retention strength 30% greater than that of the non sterilized matrix. For example, the sterilized chitosan matrix obtainable by the method of the invention has a suture retention strength at least 41%, for example from 41% to 257%, greater than the suture retention strength of a matrix of same composition sterilized by gamma irradiation.

Another aspect of the invention is also a method for increasing the suture retention strength, for example by 30% or more, of a chitosan matrix obtained by lyophilization of a chitosan solution, comprising the step of sterilizing said matrix by water vapor treatment. The matrix therefore obtained shows improved suture retention strength while being sterilized at the same time. In embodiments, the water vapor sterilization step may be an intermediate step of the manufacturing process of the matrix. Alternatively, the water vapor sterilization step may be the final step of the manufacturing process of the matrix.

Another aspect of the invention is a method for decreasing the solubilization rate in hydrochloric acid of a chitosan matrix obtained by lyophilization of a chitosan solution, comprising the step of sterilizing said matrix by water vapor treatment. The matrices obtained do not solubilize rapidly in biological fluids and are particularly suitable for use as implants or as part of composite implants.

According to a first step of the method according to the invention, step a), a solution of chitosan is prepared.

Chitosan is a biocompatible biopolymer resulting from the deacetylation of chitin. Chitin is extracted from exoskeletons of arthropods such as lobster, crab or shrimp, from the endoskeleton of cephalopods such as squid, or else from fungi. The extraction of chitin involves steps of protein and lipid hydrolysis, of depigmentation and of demineralization. Usually, the protein and lipid hydrolysis is carried out in the presence of sodium hydroxide, the demineralization requiring the use of hydrochloric acid.

Once the chitin has been extracted, the chitosan is obtained by means of a deacetylation step, which consists of hydrolysis of the acetamide groups. This reaction is generally carried out at high temperature in an alkaline solution, for example a solution of sodium hydroxide (NaOH) at 48% in water, at 90°C.

The following publications also describe methods for deacetylation of chitin in order to obtain chitosan:"Lamarque, G., C. Viton, and A. Domard, New Route of Deacetylation of α- and β-Chitins by Means of Freeze-Pump Out-Thaw Cycles. Biomacromolecules, 2005. 6(3): p. 1380-1388.", "Lamarque, G., C. Viton, and A. Domard, Comparative Study of the First Heterogeneous Deacetylation of α- and β-Chitins in a Multistep Process. Biomacromolecules, 2004. 5(3): p. 992-1001.", "Lamarque, G., C. Viton, and A. Domard, Comparative Study of the Second and Third Heterogeneous Deacetylations of α- and β-Chitins in a Multistep Process. Biomacromolecules, 2004. 5(5): p. 1899-1907.", "Tolaimate, A., et al., Contribution to the preparation of chitins and chitosans with controlled physico-chemical properties. Polymer, 2003. 44(26): p. 7939-7952."

Chitosans which may be suitable as starting materials for the method according to the invention are available from the companies Kitozyme (fungus extract), Heppe Medical chitosan (shrimp or crab extract), Primex (shrimp extract), Novamatrix (shrimp extract) and Mathani (calamari extract).

Chitosan is a biodegradable compound. The degree of acetylation (DA) of chitosan can have an influence on the chitosan degradation kinetics. Thus, depending on the biodegradation kinetics desired for the matrix prepared according to the method of the invention, and therefore for the implant formed from this matrix, the chitosan may have a degree of acetylation of 2, 3, 10, 20, 30, 40 or else 50%: the desired degree of acetylation can be obtained by preparing a mixture of several chitosans having various degrees of acetylation.

The chitosan solution of step a) of the method according to the invention is generally prepared by solubilization of chitosan in powder form in water. The solution is adjusted to the desired pH, for example by adding acetic acid. In one embodiment of the method according to the invention, the pH of the chitosan solution is adjusted to a value ranging from 3 to 5, preferably to 3.5, for example by adding acetic acid. Such a pH makes it possible to obtain, in the end, a chitosan matrix which has good mechanical properties for example.

In one embodiment of the method according to the invention, the solution of step a) is a solution of chitosan in water, the chitosan concentration in said solution ranging from 0.1% to 10%, preferably from 0.5% to 3% and more preferably from 0.8% to 1% by weight, relative to the total weight of the solution.

Such chitosan concentrations make it possible to obtain, in the end, matrices which are particularly resistant to suturing. Also preferably, the chitosan solution of the method according to the invention is free of any other polymer than the chitosan. The presence of a polymer other than chitosan in the solution could affect the mechanical or biological properties of the matrices.

According to a second step of the method according to the invention, step b), the solution of chitosan is poured into a mould in order to form a layer. For example, the mould is in the shape of a rectangle having dimensions compatible with the use of the matrix obtained as all or part of a reinforcing implant for the abdominal wall or of a haemostatic patch.

According to a third step of the method according to the invention, step c), the layer obtained in b) is lyophilized so as to obtain a matrix.

In the present application, the term "matrix" is intended to mean a layer which has pores, or gaps, alveoli, holes, orifices, which are evenly or unevenly distributed not only at the surface, but also within the thickness of said layer, and which are more or less interconnected, depending on the lyophilization process used. Such lyophilization processes are known. It is known practice to vary the freezing temperature and speed and also the characteristics of the polymer solution to be lyophilized (pH, concentration, etc.), in this case the chitosan solution, as a function of the structure of the matrix that it is desired to obtain (see US 4 970 298; Doillon et al, J Biomed Mater Res, 1986; Schoof, J Biomed Mater Res, 2001; O'Brien et al, Biomaterials, 2004).

According to a fourth step of the method according to the invention, step d), the matrix obtained in c) is sterilized by water vapor treatment.

The matrix is placed in an autoclave. The air present in the autoclave is evacuated and water vapor is introduced at a determined temperature and during a specified time. For example, the water vapor treatment may be completed in conformity with the B-process of European standard EN 13060.

The temperature and the pressure within the autoclave are controlled during the time of the treatment.

In embodiments, the matrix is is treated with water vapor for 20 min at 121 °C.

In other embodiments, the matrix is treated with water vapor for 4 min at 134°C.

In other embodiments, the matrix is treated with water vapor for 75 min at 134°C. Matrices treated with water vapor for 75 min at 134°C show in particular a very slow solubilization rate in hydrochloric acid.

The water vapor treatment allows killing microorganisms by destroying metabolic and structural components essential to their replication. The treated matrix is therefore sterilized.

This water vapor sterilization step has also as a consequence that the suture retention strength of the matrix is significantly increased with respect to the non sterilized matrix, or with respect to matrices sterilized by another sterilization method. In addition, the water vapor sterilized matrices show a decrease in their solubilization rate in hydrochloric acid.

The sterilized chitosan matrix obtained according to the method of the invention can be part of, or constitute, the whole of a surgical implant, for example an abdominal wall reinforcement or of an haemostatic patch. Indeed, these sterilized matrices have excellent suture retention strength.

Another aspect of the invention relates to a surgical implant, such as an abdominal wall reinforcement, for example a hernia repair implant, or a haemostat such as a haemostatic patch, comprising at least one chitosan matrix obtained by the method described above. An aspect of the invention relates to a hernia repair implant comprising at least one chitosan matrix obtained by the method described above. For example, the hernia repair implant may consist in a chitosan matrix obtained by the method described above. An aspect of the invention relates to a haemostat comprising at least one chitosan matrix obtained by the method described above. For example, the haemostat may consist in a chitosan matrix obtained by the method described above.

The invention and the advantages thereof will emerge more clearly from the examples below.

### EXAMPLE 1 :

### 1°) Production of chitosan matrices :

Matrices of chitosan of DA (degree of acetylation) 4%, and of chitosan of DA 50%, were produced according to the following procedure : chitosan was first suspended in water and dissolved under mechanical stirring by adding acetic acid to achieve the stoechiometric protonation of free amine groups of D-glucosamine residues. Usually, an excess of 10% of acetic acid is added to enhance the dissolution.

This operation is realized in a STEPHAN unit with mechanical stirring at 300 rpm and room temperature. To avoid any temperature increase, the bowl is equipped with a cooling bath to maintain the room temperature at a temperature T<25°C. The dissolution takes about 2 to 3 hours in these conditions.

After complete dissolution of the polymer, the pH of the chitosan solution is adjusted to pH 3.5 by addition of acetic acid. Solutions of pH 3.5 allow improving the mechanical properties of resulting matrices. The solution (typically 0.8% or 1% (w/w) chitosan solution) is then degassed under static vacuum : such a step allows removing air bubbles that could impact both porosity and mechanical properties of the resulting matrices. For example, the solution is degassed at 150 mbars, 300 rpm and 25°C for 30 min. 2 or 3 degassing cycles are usually necessary to achieve complete degassing.

121 g of degassed chitosan solution are then poured in a plastic mould of 12 cm X17cm dimensions in order to form a layer which is freeze-dried in a TELSTAR Lyobeta freeze drier according to a 28 hours cycle, namely a freezing step of about 4 hours and a primary drying step of about 24 hours.

The following matrices were thus produced:
- 3 matrices of chitosan DA4%: Solution of 0.8 % (w/w) chitosan. 3.73 g of chitosan are dissolved in 1.39 mL AcOH, and 446 g sterile water. The initial pH of the solution is 5.12. The pH of the solution is then adjusted to 3.51 by addition of 11.0 mL of AcOH.
- 3 matrices of chitosan DA50%: Solution of 1.0% (w/w) chitosan. 4.70 g of chitosan are dissolved in 1.56 mL AcOH, and 445 g sterile water. The initial pH of the solution is 4.50. The pH of the solution is adjusted to 3.50 by addition of 7.5 mL of AcOH.

### 2°) Neutralization of the chitosan matrices produced at 1°):

The matrices obtained at point 1°) above were neutralized during 5 minutes into a solution of NaOH 0.5M in pure ethanol : each matrix was dipped in about 600-800 mL of neutralizing solution.The matrices were then washed in sterile water baths during 10 min (typically 1 L/matrix) to remove the excess of sodium hydroxide and residual salts. Washings were repeated until the washing solution reached a neutral pH and a conductivity below 2 µS/cm. After washings, the matrices were loaded in plastic moulds and freeze-dried in a TELSTAR Lyobeta freeze drier according to a 20 hours cycle, namely a freezing step of about 2 hours and a primary drying step of about 18 hours. After drying, they were packed in individual Tyvek® bags. The packed matrices were then submitted to water vapor sterilization as described in point 3°) below.

### 3°) Water vapor sterilization treatment of matrices obtained at 2°):

A part of the matrices obtained at point 2°) above were sterilized with water vapor for 4 min at 134°C or for 75 min at 134°C (Matrices of the invention). The other part of the matrices obtained at point 2°) above were left unsterilized (Comparative matrices).

### 4°) Solubilization in hydrochloric acid :

The comparative matrices matrices dissolved over night in a 0.5%HCl (12M) solution in D₂0 while the matrices of the invention did not. The matrices of the invention needed much longer time to solubilize. For some of the matrices of the invention, heating at 60°C or ultra sonic treatment was needed to reach solubilization.

The matrices of the invention that had been treated at 134°C for 75 min were harder to solubilize and remained intact for a long time even at rigorous stirring. No change of chemical structure was shown by NMR analysis, *i.e.* DA was not modified.

These results show that sterilizing chitosan matrices with water vapor treatment decreases the solubilization rates of such matrices in hydrochloric acid. Water vapor sterilized chitosan matrices show slower solubilization rates than unsterilized matrices of the same chitosan composition.

The matrices of the invention of the present example may be used directly as implants, or may be used in the manufacture of composite implants. The implants formed of, or comprising, the matrices of the invention do not solubilize rapidly in biological fluids and are capable of performing their function, for example haemostatic function or reinforcement function, during a significant time.

### EXAMPLE 2 :

### 1°) Production of neutralized chitosan matrices :

Matrices of chitosan of DA 2% were prepared according to the following procedure : 0.8% (w/w) chitosan solutions adjusted at pH 3.5 with acetic acid were freeze-dried (121 g of solution in 12X17 cm plastic mould). Resultant matrices were neutralized for 5 min in a NaOH 0.5M in water solution and then washed in sterile water baths.

### 2°) Water vapor sterilization treatment of matrices obtained at 1°) :

A part of the matrices obtained at point 1°) above of the present example were sterilized with water vapor for 20 min at 121°C (Matrices A, according to the invention) or 4 min at 134°C (Matrices B, according to the invention).

Another part of the matrices obtained at point 1°) above of the present example were left unsterilized (Matrices C, comparative).

Another part of the matrices obtained at point 1°) above of the present example were sterilized with ethylene oxide (Matrices D, comparative).

A last part of the matrices obtained at point 1°) above of the present example were sterilized by gamma irradiation (Matrices E, comparative).

### 3°) Suture retention strength :

Matrices A and B of the invention and comparative matrices C, D and E were then submitted to the suture retention testing as described in French standard NF 594-801. This testing consists in introducing a suture yarn such as Surgipro® II 5-0 in a square-shaped sample matrix moistened beforehand. The suture yarn is the drawn at a speed of 50 mm/min until complete breakage. The suture retention strength corresponds to the measured force at the time of breakage.

The results are collated in the Table I below : 6 tests were performed for each matrix.

**Table I : Suture retention strength of Matrices A-E**

| | Suture retention strength in N | | | | |
|---|---|---|---|---|---|
| | Matrix A (invention) | Matrix B (invention) | Matrix C (comparative) | Matrix D (comparative) | Matrix E (comparative) |
| Test 1 | 1.67 | 1.69 | 1.21 | 1.10 | 0.60 |
| Test 2 | 1.58 | 1.85 | 1.43 | 1.54 | 0.76 |
| Test 3 | 1.64 | 1.90 | 1.42 | 1.12 | 0.93 |
| Test 4 | 1.31 | 1.42 | 1.31 | 1.23 | 0.82 |
| Test 5 | 2.14 | 1.49 | 1.06 | 0.98 | 0.88 |
| Test 6 | 1.50 | 1.53 | 1.18 | 1.19 | 0.62 |
| ***Average*** | ***1.64*** | ***1.65*** | ***1.27*** | ***1.19*** | ***0.77*** |

These results show that the matrices A and B of the invention show a significant increase of the suture retention strength compared to comparative unsterilized matrices C, comparative ethylene oxide-sterilized matrices D or comparative gamma irradiated matrices E.

By comparing the average values for each matrix, one can see that the suture retention strength of the matrices of the invention is increased by about 30% or more compared to suture retention strength of unsterilized matrices.

For each test (Tests 1-6), the improvement in % of the suture retention strength of Matrix A of the invention versus the suture retention strength measured for each test for comparative Matrix E sterilized by gamma irradiation was calculated. The results are collated in Table II below.

**Table II : Improvement in % of suture retention strength of Matrix A vs Matrix E for each Test 1-6.**

| | **Improvement of suture retention strength of Matrix A vs Matrix E** | | | | | |
|---|---|---|---|---|---|---|
| **Matrix E** | **Matrix A Test 1** | **Matrix A Test 2** | **Matrix A Test 3** | **Matrix A Test 4** | **Matrix A Test 5** | **Matrix A Test 6** |
| **Test 1** | 179% | 165% | 174% | 119% | 257% | 152% |
| **Test 2** | 121% | 110% | 117% | 74% | 183% | 99% |
| **Test 3** | 80% | 71% | 77% | 41% | 130% | 62% |
| **Test 4** | 103% | 92% | 100% | 59% | 160% | 83% |
| **Test 5** | 91% | 81% | 87% | 50% | 144% | 72% |
| **Test 6** | 170% | 156% | 165% | 112% | 245% | 143% |

The suture retention strength of matrices of the invention sterilized with water vapor for 20 min at 121°C (Matrices A) is increased by at least 41% with respect to that of matrices of the same composition sterilized with gamma irradiation. Indeed, the suture retention strength of matrices of the invention sterilized with water vapor for 20 min at 121°C (Matrices A) is increased up to 257% with respect to that of matrices of the same composition sterilized with gamma irradiation.

For each test (Tests 1-6), the improvement in % of the suture retention strength of Matrix B of the invention versus the suture retention strength measured for each test for comparative Matrix E sterilized by gamma irradiation was calculated. The results are collated in Table III below.

**Table III : Improvement in % of suture retention strength of Matrix B vs Matrix E for each Test 1-6.**

| | **Improvement of suture retention strength of Matrix B vs Matrix E** | | | | | |
|---|---|---|---|---|---|---|
| **Matrix E** | **Matrix B Test 1** | **Matrix B Test 2** | **Matrix B Test 3** | **Matrix B Test 4** | **Matrix B Test 5** | **Matrix B Test 6** |
| **Test 1** | 183% | 145% | 217% | 138% | 149% | 157% |
| **Test 2** | 124% | 99% | 151% | 88% | 97% | 103% |
| **Test 3** | 83% | 125% | 105% | 53% | 61% | 65% |
| **Test 4** | 106% | 111% | 131% | 73% | 81% | 87% |
| **Test 5** | 93% | 199% | 117% | 62% | 70% | 75% |
| **Test 6** | 174% | 141% | 207% | 130% | 141% | 148% |

The suture retention strength of matrices of the invention sterilized with water vapor for 4 min at 134°C (Matrices B) is increased by at least 53% with respect to that of matrices of the same composition sterilized with gamma irradiation. Indeed, the suture retention strength of matrices of the invention sterilized with water vapor for 4 min at 134°C (Matrices B) is increased up to 217% with respect to that of matrices of the same composition sterilized with gamma irradiation.

Matrices of the invention have a suture retention strength at least 41%, for example from 41% to 257%, greater than the suture retention strength of a matrix of same composition sterilized by gamma irradiation.

The water vapor treatment provides a method to improve mechanical and physical properties of chitosan matrices. Another advantage is that the treatment can serve as a sterilization method at the same time.

## Claims

1. Method for preparing a chitosan matrix having good mechanical properties, in particular good suture retention strength, comprising the steps of:
- a) preparing a solution of chitosan,
- b) pouring the solution of a) into a mould in order to form a layer,
- c) lyophilizing the layer of b) in order to obtain a matrix of chitosan,
- d) sterilizing the matrix obtained in c) by water vapor treatment.

2. Method according to claim 1, in which the solution of step a) is a solution of chitosan in water, the chitosan concentration in said solution ranging from 0.1 to 10%, preferably from 0.5% to 3%, and more preferably from 0.8 to 1 % by weight, relative to the total weight of the solution.

3. Method according to claim 1 or 2, in which the pH of the solution in a) is adjusted to a value ranging from 3 to 5, preferably to 3.5, for example by adding acetic acid.

4. Method according to any one of claims 1 to 3, in which the solution of step a) is free of any other polymer than the chitosan.

5. Method according to any one of claims 1-4, wherein, in step d), the matrix is treated with water vapor for 20 min at 121 °C.

6. Method according to any one of claims 1-4, wherein, in step d), the matrix is treated with water vapor for 4 min at 134°C.

7. Method according to any one of claims 1-4, wherein, in step d), the matrix is treated with water vapor for 75 min at 134°C.

8. Sterilized chitosan matrix obtainable by the method of any one of claims 1 to 7.

9. Sterilized chitosan matrix according to claim 8, having a suture retention strength at least 41%, for example from 41% to 257%, greater than the suture retention strength of a matrix of same composition sterilized by gamma irradiation.

10. Surgical implant comprising at least one sterilized matrix of chitosan according to any one of claims 8-9.

11. Hernia repair implant comprising at least one sterilized matrix of chitosan according to any one of claims 8-9.

12. Haemostat comprising at least one sterilized matrix of chitosan according to any one of claims 8-9.

13. Method for increasing the suture retention strength, for example by 30% or more, of a chitosan matrix obtained by lyophilization of a chitosan solution, comprising the step of sterilizing said matrix by water vapor treatment.

14. Method for decreasing the solubilization rate in hydrochloric acid of a chitosan matrix obtained by lyophilization of a chitosan solution, comprising the step of sterilizing said matrix by water vapor treatment.

15. Method of claim 14 or 15, wherein the matrix is treated with water vapor for 20 min at 121 °C.

16. Method according to claim 14 or 15, wherein the matrix is treated with water vapor for 4 min at 134°C.

17. Method according to claim 14 or 15, wherein the matrix is treated with water vapor for 75 min at 134°C.
